(19) Europäisches Patentamt
European Patent Office
Office européen des brevets

(11) Veröffentlichungsnummer: **0 545 149 B1**

(12) # EUROPÄISCHE PATENTSCHRIFT

(45) Veröffentlichungstag der Patentschrift: **07.09.94**

(51) Int. Cl.$^{5}$: **C07D 251/16**

(21) Anmeldenummer: **92119689.5**

(22) Anmeldetag: **19.11.92**

(54) **Verfahren zur Herstellung von Derivaten des 6-Trifluormethyl-1,3,5-triazins.**

(30) Priorität: **30.11.91 DE 4139624**

(43) Veröffentlichungstag der Anmeldung:
**09.06.93 Patentblatt 93/23**

(45) Bekanntmachung des Hinweises auf die Patenterteilung:
**07.09.94 Patentblatt 94/36**

(84) Benannte Vertragsstaaten:
**AT BE CH DE ES FR GB IT LI NL**

(56) Entgegenhaltungen:
**EP-A- 0 482 477**
**DE-A- 1 670 147**

**CHEMICAL ABSTRACTS, vol. 83, 1975, Columbus, Ohio, US; abstract no. 164133k, TSUJIKAWA, TERUAKU ET AL. 'Hetrocyclic compounds. I. Syntheses of 1,3,5-triazine derivatives and their pharmacological activities' Seite 550 ;**

(73) Patentinhaber: **BASF Aktiengesellschaft**
**Carl-Bosch-Strasse 38**
**D-67063 Ludwigshafen (DE)**

(72) Erfinder: **Mayer, Horst, Dr.**
**Faselwiese 19**
**W-6700 Ludwigshafen (DE)**
Erfinder: **Hamprecht, Gerhard, Dr.**
**Rote-Turm-Strasse 28**
**W-6940 Weinheim (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft ein neues Verfahren zur Herstellung von Derivaten des 6-Trifluormethyl-1,3,5-triazins der allgemeinen Formel I

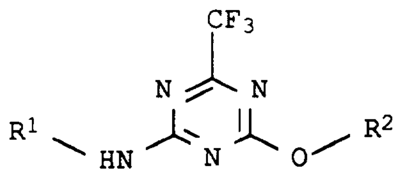

I

in der $R^1$ Wasserstoff oder einen Kohlenwasserstoffrest, der gegebenenfalls mit unter den Reaktionsbedingungen inerten Substituenten substituiert sein kann und $R^2$ einen Kohlenwasserstoffrest, der gegebenenfalls mit unter den Reaktionsbedingungen inerten Substituenten substituiert sein kann, bedeuten.

Weiterhin betrifft die Erfindung ein neues Verfahren zur Herstellung von Derivaten des 6-Trifluormethyl-1,3,5-triazins der allgemeinen Formel V

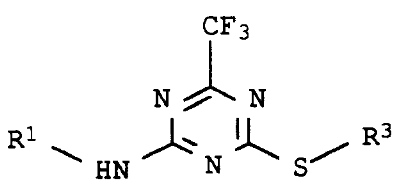

V

in der $R^1$ Wasserstoff oder einen Kohlenwasserstoffrest, der gegebenenfalls mit unter den Reaktionsbedingungen inerten Substituenten substituiert sein kann und $R^3$ einen Kohlenwasserstoffrest mit 1 bis 10 C-Atomen, der gegebenenfalls mit unter den Reaktionsbedingungen inerten Substituenten substituiert sein kann, bedeuten, die als Zwischenprodukte für die Herstellung von I dienen.

Die DE-A 16 70 147 betrifft die Umsetzung von N-Amidino-S-alkylisothiuronium-Salzen mit Carbonsäureacyl-Äquivalenten zu 1,3,5-Triazinen. Die hiernach erzielten hohen Ausbeuten von 80 - 100 % werden jedoch nur bei Cyclisierungsreaktionen mit aromatischen, unpolaren Carbonsäurechloriden erreicht. Die Synthese von 2-Amino-4-methylthio-6-trichlormethyl-1,3,5-triazin aus dem aliphatischen, polareren Trichloracetylchlorid und N-Amidino-S-methylisothioharnstoff verläuft in Gegenwart von Triethylamin dagegen nur mit 60 % Ausbeute.

6-Trichlormethylsubstituierte 1,3,5-Triazine, die in 2-Stellung einen Amino- und in 4-Stellung einen Thio-Substituenten tragen, sind weiterhin nach der JB-A 48038715 in einer zweistufigen Umsetzung zugänglich. Hierbei wird zunächst 2-Methylthio-4,6-bis(trichlormethyl)-1,3,5-triazinhergestellt, welches dann mit einem entsprechenden Amin unter Freisetzung von Chloroform zum Endprodukt umgesetzt wird.

2-Alkoxy-4-amino-6-trifluormethyl-1,3,5-triazine sind nach einem aus Yakugaku Zasshi 95 (1975) 499 bekannten Verfahren erhältlich. Hierbei wird N-Cyanguanidin in einen Bis (N-amidino-O-alkylisoharnstoff) kupfer-Chelatkomplex überführt, aus dem der Ligand durch Behandlung mit Schwefelwasserstoff freigesetzt und anschließend mit einem Trifluoressigsäureester zum Endprodukt cyclisiert wird.

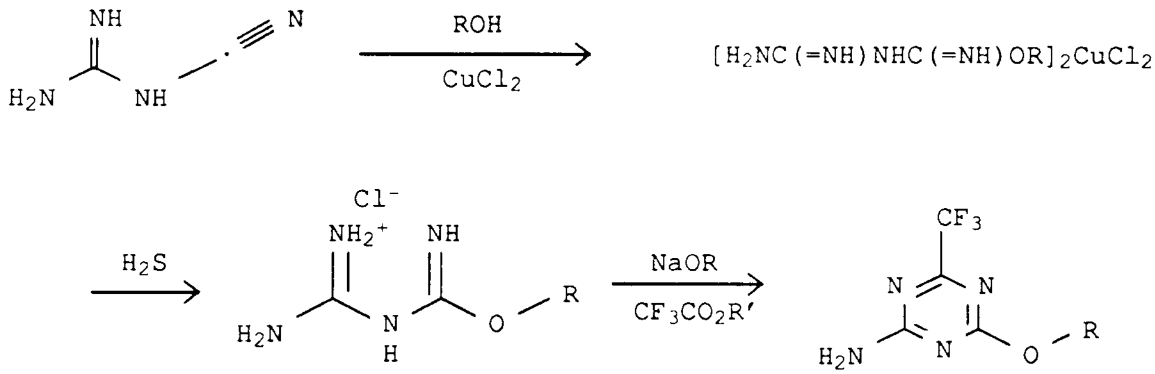


R,R' = organische Reste

Für dieses Verfahren sind stöchiometrische Mengen von Cu-Salzen nötig; in Abwesenheit von Cu-Salzen wird statt des N-Amidino-O-alkylisoharnstoffs hauptsächlich Guanylharnstoff gebildet (Kyushu Kogyo Daigaku Kenkyu Hokoku No. 12, 69-78 (1962).

Ein großer Nachteil dieses Verfahrens besteht darin, daß große Mengen an Kupfersalzen als Nebenprodukt abgetrennt und entsorgt werden müssen, was eine Durchführung im technischen Maßstab unrentabel erscheinen läßt.

In Zh. Obshch. Khim. 1967, 37, 2247-51 wird die nukleophile Substitution an 1,3,5-Triazinen beschrieben, wobei ein elektronegativer Substituent durch einen weniger elektronegativen ersetzt werden kann. Es wird jedoch keine Aussage über das Verhalten von 6-Trifluormethyl-1,3,5-triazinen gemacht.

Der Erfindung lag daher die Aufgabe zugrunde, Derivate des 6-Trifluormethyl-1,3,5-triazins auf vorteilhaftere Weise zugänglich zu machen.

Demgemäß wurde ein Verfahren zur Herstellung von Derivaten des 6-Trifluormethyl-1,3,5-triazins der allgemeinen Formel I,

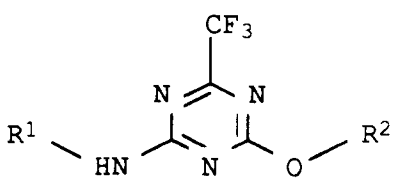

I

in der $R^1$ Wasserstoff oder einen Kohlenwasserstoffrest, der gegebenenfalls mit unter den Reaktionsbedingungen inerten Substituenten substituiert sein kann und $R^2$ einen Kohlenwasserstoffrest, der gegebenenfalls mit unter den Reaktionsbedingungen inerten Substituenten substituiert sein kann, bedeuten, gefunden, welches dadurch gekennzeichnet ist, daß man ein Salz eines N-Amidino-S-alkylisothioharnstoffs der allgemeinen Formel II

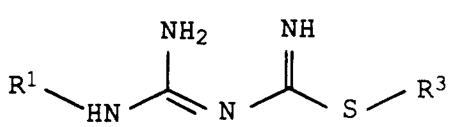

II

in der $R^3$ für einen Kohlenwasserstoffrest mit 1 bis 10 C-Atomen, der gegebenenfalls mit unter den Reaktionsbedingungen inerten Substituenten substituiert sein kann, steht, mit einem Halogenid, Ester oder Anhydrid der Trifluoressigsäure (Verbindung III) sowie mit einer starken Base (IV) zu einem Derivat des 6-Trifluormethyl-1,3,5-triazins der allgemeinen Formel V

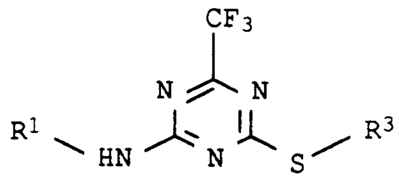

V

umsetzt und dieses in Gegenwart einer Base (VI) mit einem Alkohol der allgemeinen Formel VII

$R^2$-OH VII

in die Verbindung I überführt.

Die als Ausgangsstoffe dienenden Salze der N-Amidino-S-alkylisothioharnstoffe II sind bekannt (Chem. Ber. 100 (1967) 1874) oder lassen sich mit Hilfe der dort beschriebenen Verfahren aus bekannten Stoffen herstellen.

Mit Salzen der N-Amidino-S-alkylisothioharnstoffe sind die Reaktionsprodukte der Verbindungen II mit Säuren gemeint. Die Art der Säure spielt für das erfindungsgemäße Verfahren keine Rolle, da im Verlauf der Reaktion die Verbindungen II aus ihren Salzen mit Hilfe einer Base freigesetzt werden. Die Verbindungen II oder ihre Salze werden zweckmäßigerweise als Addukte an N-Methyl-2-pyrrolidon hergestellt bzw. aufbewahrt.

Nach den bisherigen Beobachtungen ist das gute Gelingen des erfindungsgemäßen Verfahrens von der Natur der Substituenten $R^1$, $R^2$ und $R^3$ nicht erkennbar abhängig.

Im Hinblick auf die Verwendung der Verbindungen I als Zwischenprodukte für die Synthese von Wirkstoffen hat der Rest $R^1$ vorzugsweise folgende Bedeutung:

- Wasserstoff;
- aliphatische Reste, mit 1 bis 6 C-Atomen wie $C_1$-$C_6$-Alkylgruppen, darunter vor allem der Methyl-, Ethyl-, n-Propyl-, Isopropyl-, n-Butyl-, sec.-Butyl- oder tert.-Butylrest oder Cycloalkylgruppen mit bis zu 7 C-Atomen wie Cyclopropyl, Cyclopentyl oder Cyclohexyl;
- aromatische Reste wie die Phenylgruppe.

$R^2$ und $R^3$ haben vorzugsweise folgende Bedeutung:

- eine $C_1$-$C_4$-Alkylgruppe wie Methyl, Ethyl, n-Propyl, Isopropyl, n-Butyl, sec.-Butyl oder tert.-Butyl;
- eine $C_3$-$C_4$-Alkenylgruppe wie Prop-2-en-1-yl, 1-Methylprop-2-en-1-yl, But-2-en-1-yl oder But-3-en-1-yl;
- eine $C_3$-$C_4$-Alkinylgruppe wie Prop-2-in-1-yl oder But-2-in-1-yl;
- eine $C_3$-$C_6$-Cycloalkylgruppe wie Cyclopropyl, Cyclobutyl, Cyclopentyl oder Cyclohexyl, bevorzugt Cyclopentyl oder Cyclohexyl.

Der Rest $R^3$ bedeutet vorzugsweise einen der für $R^2$ genannten Reste oder besonders die unsubstituierte oder mit Methyl, Chlor, Brom oder Methoxy kernsubstituierte Benzylgruppe.

Die genannten Reste $R^1$ bis $R^3$ können noch unter den Reaktionsbedingungen inerte Substituenten tragen.

Als Verbindung III eignen sich vornehmlich Trifluoressigsäureanhydrid, Halogenide der Trifluoressigsäure und die Ester der Trifluoressigsäure, wobei der Methyl- und der Ethylester ganz besonders bevorzugt sind.

Für die Herstellung der 4-Thioalkyl-6-trifluormethyl-1,3,5-triazine V aus II und III, empfiehlt sich eine mindestens äquimolare Menge an der Verbindung III; bevorzugt werden Mengen von 100 bis 500 mol-%, insbesondere von 100 bis 250 mol-% an Trifluoressigsäurederivat III bezogen auf II, sofern III nicht auch als Lösungsmittel verwendet wird.

Als Basen IV eignen sich anorganische und organische Basen. Die Stärke der eingesetzten Base soll so groß sein, daß sie den N-Amidino-S-alkylisothioharnstoff II aus seinem Salz freisetzen kann.

Als anorganische Basen werden Alkalimetall- und Erdalkalimetallhydroxide bevorzugt, und als organische Basen Amine und Alkalimetallalkoholate.

Die Menge an Base beträgt üblicherweise 110 bis 300 mol-% der Menge des eingesetzten Salzes des N-Amidino-S-alkylisothioharnstoffs II. Größere Mengen sind möglich, bringen in der Regel aber keine weiteren Vorteile. Bei der Reaktionsführung mit einem Trifluoressigsäureester sind 2-3 Äquivalente an Base, bezogen auf das Salz von II, besonders bevorzugt.

Zweckmäßigerweise legt man das Salz von II und die Verbindung III vor und dosiert die Base zu.

Vorzugsweise arbeitet man in einem inerten Lösungs- bzw. Verdünnungsmittel bei der Umsetzung von II mit III.

Als Lösungsmittel eignen sich beispielsweise aliphatische oder aromatische Kohlenwasserstoffe, die auch durch Halogen oder eine Nitrogruppe substituiert sein können, cyclische oder offenkettige Ether, aliphatische Alkohole oder niedere aliphatische Ketone. Auch Säurederivate von niederen Carbonsäuren wie Essigsäureester, Formamide oder Nitrile sind gut als Lösungsmittel geeignet.

Führt man die Umsetzung von II mit Trifluoressigsäureanhydrid oder Trifluoressigsäurechlorid durch, so sind Ethylacetat, Diethylether und tert.Butylmethylether als Lösungsmittel besonders bevorzugt.

Bei Reaktionsführung mit einem Trifluoressigsäureester verwendet man besonders bevorzugt Methanol, Tetrahydrofuran, tert.Butylmethylether oder den Trifluoressigester selbst als Lösungsmittel.

Die Menge an Lösungsmittel ist nicht kritisch. Normalerweise verwendet man die 1- bis 5-fache Menge an Lösungsmittel, bezogen auf die Menge an N-Amidino-S-alkylisothioharnstoff II.

Besondere Bedingungen bezüglich des Druckes sind nicht erforderlich, im allgemeinen nimmt man die Umsetzung bei Atmosphärendruck vor.

Die Umsetzung wird zweckmäßigerweise bei Temperaturen zwischen -40 °C und der Siedetemperatur des Lösungsmittels, bevorzugt zwischen -20 °C und 120 °C, insbesondere zwischen -10 °C und 100 °C durchgeführt.

Sie kann sowohl kontinuierlich als auch diskontinuierlich durchgeführt werden. Bei der kontinuierlichen Arbeitsweise leitet man die Reaktionspartner vorzugsweise durch einen Rohrreaktor.

Die Aufarbeitung des Reaktionsgemisches erfolgt allgemein in der Weise, daß man die niedrigsiedenden Bestandteile unter reduziertem Druck entfernt, Säurereste neutralisiert und die anorganischen Bestandteile durch Verrühren des Rohproduktes mit Wasser herauslöst.

Die Substitution der Thioalkylgruppe des 6-Trifluormethyl-1,3,5-triazins V durch eine Alkoxygruppe wird zweckmäßigerweise in einem Überschuß des Alkohols VII durchgeführt.

Als Base VI eignen sich anorganische und organische Basen. Als anorganische Basen kommen bevorzugt Hydroxide und Carbonate von Alkalimetall-, Erdalkalimetall- oder Aluminiumionen, als organische Basen Amine und Alkalimetallalkoholate in Betracht.

Besonders bevorzugt verwendet man die Alkalimetallalkoholate des jeweiligen Alkohols VII.

Die Menge an Base VI beträgt im allgemeinen 1 bis 200 mol-%, bezogen auf die Menge an 6-Trifluormethyl-1,3,5-triazin V. Bevorzugt verwendet man 50 bis 150 mol-% an Base bei $C_1$- und $C_2$-Alkoholen VII und über 50 mol-% bei Verbindungen VII mit 3 und mehr Kohlenstoffatomen.

Die Menge an Lösungsmittel ist nicht kritisch. Normalerweise verwendet man die 5- bis 10-fache Menge an Lösungsmittel, bezogen auf die Menge an 6-Trifluormethyl-1,3,5-triazin V, wobei man vorzugsweise als Lösungsmittel einen Überschuß an Alkohol VII verwendet.

Die Menge an Alkohol der Formel VII muß für eine vollständige Umsetzung mindestens äquimolar zur eingesetzten Menge an 6-Trifluormethyl-1,3,5-triazin V sein. Arbeitet man ohne zusätzliche Lösungsmittel, so werden bevorzugt 4 bis 5 mol des Alkohols V pro mol an 6-Trifluormethyl-1,3,5-triazin V verwendet.

Man führt die Umsetzung von V mit VII in der Regel bei Temperaturen zwischen -40 °C und der Siedetemperatur des jeweiligen Lösungsmittels, bevorzugt zwischen -30 °C und 150 °C, insbesondere zwischen -10 und 80 °C durch, besonders bevorzugt zwischen 0 bis 50 °C.

Die Aufarbeitung des Reaktionsgemisches erfolgt wie üblich, und zwar in der Regel durch Entfernen der niedrigsiedenden Bestandteile bei reduziertem Druck nach Neutralisierung.

Die Umsetzung von V mit VII kann sowohl kontinuierlich als auch diskontinuierlich durchgeführt werden. Bei der kontinuierlichen Arbeitsweise leitet man die Reaktionspartner beispielsweise über ein Festbett aus einer unlöslichen Base, oder man führt die Reaktion in einer mit dem Produkt I gesättigten Lösung unter laufender Entfernung von neu gebildetem Produkt durch.

Eine Variante des erfindungsgemäßen Verfahrens besteht darin, das Verfahrensprodukt V aus der Umsetzung von II mit III ohne Isolierung aus der Reaktionsmischung gleich mit VI und VII umzusetzen, wobei saure Nebenprodukte aus der ersten Umsetzung durch eine höhere Konzentration an Base VI neutralisiert werden können. Die Verfahrensweise kann dahingehend abgewandelt werden, daß man nach Durchführung der Umsetzung von II mit III die niedrigsiedenden Bestandteile entfernt und das erhaltene Rohprodukt, gewünschtenfalls in einem anderen Lösungsmittel, mit VI und VII umsetzt.

Das erfindungsgemäßen Verfahren (Umsetzung von V mit VII), läßt sich mit Erfolg zur Synthese aller definitonsgemäßen 6-Trifluormethyl-1,3,5-triazine I anwenden, vor allem der in Tabelle 1 enthaltenen Verbindungen. Die 6-Trifluormethyl-1,3,5-triazine I sind wertvolle Zwischenprodukte für Pflanzenschutzmittel, wie sie beispielsweise aus der EP-A 111 442 oder der DE-A 39 09 146 bekannt sind.

Tabelle 1

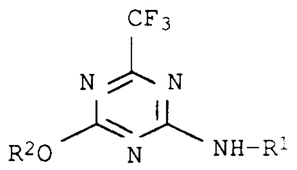

| $R^1$ | $R^2$ |
|---|---|
| H | $CH_3$ |
| H | $C_2H_5$ |
| H | $n-C_3H_7$ |
| H | $i-C_3H_7$ |
| H | $n-C_4H_9$ |
| H | $i-C_4H_9$ |
| H | $s-C_4H_9$ |
| H | $t-C_4H_9$ |
| H | $CH_2CH=CH_2$ |
| H | $E-CH_2CH=CHCH_3$ |
| H | $CH_2C\equiv CH$ |
| H | $CH_2C\equiv CCH_3$ |
| H | Cyclopropyl |
| H | Cyclobutyl |
| H | Cyclopentyl |
| H | Cyclohexyl |
| $CH_3$ | $CH_3$ |
| $CH_3$ | $C_2H_5$ |
| $CH_3$ | $n-C_3H_7$ |
| $CH_3$ | $i-C_3H_7$ |
| $CH_3$ | $n-C_4H_9$ |
| $CH_3$ | $i-C_4H_9$ |
| $CH_3$ | $s-C_4H_9$ |
| $CH_3$ | $t-C_4H_9$ |
| $CH_3$ | $CH_2CH=CH_2$ |
| $CH_3$ | $E-CH_2CH=CHCH_3$ |
| $CH_3$ | $CH_2C\equiv CH$ |
| $CH_3$ | $CH_2C\equiv CCH_3$ |
| $CH_3$ | Cyclopropyl |

| $R^1$ | $R^2$ |
|---|---|
| $CH_3$ | Cyclobutyl |
| $CH_3$ | Cyclopentyl |
| $CH_3$ | Cyclohexyl |
| $C_2H_5$ | $CH_3$ |
| $C_2H_5$ | $C_2H_5$ |
| $C_2H_5$ | $n-C_3H_7$ |
| $C_2H_5$ | $i-C_3H_7$ |
| $C_2H_5$ | $n-C_4H_9$ |
| $C_2H_5$ | $i-C_4H_9$ |
| $C_2H_5$ | $s-C_4H_9$ |
| $C_2H_5$ | $t-C_4H_9$ |
| $C_2H_5$ | $CH_2CH=CH_2$ |
| $C_2H_5$ | $E-CH_2CH=CHCH_3$ |
| $C_2H_5$ | $CH_2C≡CH$ |
| $C_2H_5$ | $CH_2C≡CCH_3$ |
| $C_2H_5$ | Cyclopropyl |
| $C_2H_5$ | Cyclobutyl |
| $C_2H_5$ | Cyclopentyl |
| $C_2H_5$ | Cyclohexyl |
| $n-C_3H_7$ | $CH_3$ |
| $i-C_4H_9$ | $C_2H_5$ |
| $n-C_3H_7$ | $n-C_3H_7$ |
| $i-C_4H_9$ | $i-C_3H_7$ |
| $n-C_3H_7$ | $n-C_4H_9$ |
| $s-C_4H_9$ | $i-C_4H_9$ |
| $i-C_3H_7$ | $s-C_4H_9$ |
| $t-C_4H_9$ | $t-C_4H_9$ |
| $i-C_3H_7$ | $CH_2CH=CH_2$ |
| $t-C_4H_9$ | $E-CH_2CH=CHCH_3$ |
| $i-C_3H_7$ | $CH_2C≡CH$ |
| $t-C_4H_9$ | $CH_2C≡CCH_3$ |
| $i-C_3H_7$ | Cyclopropyl |
| $t-C_4H_9$ | Cyclobutyl |
| $i-C_3H_7$ | Cyclopentyl |
| $t-C_4H_9$ | Cyclohexyl |

Das erfindungsgemäße Verfahren (Umsetzung von II mit III) läßt sich mit Erfolg zur Synthese aller definitionsgemäßen 6-Trifluormethyl-1,3,5-triazine V anwenden, vor allem der in Tabelle 2 benannten Verbindungen.

Tabelle 2

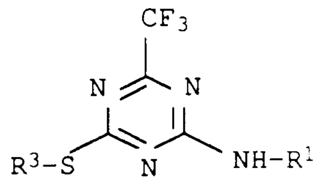

| $R^1$ | $R^3$ |
|---|---|
| H | $CH_3$ |
| H | $C_2H_5$ |
| H | $n-C_3H_7$ |
| H | $i-C_3H_7$ |
| H | $n-C_4H_9$ |
| H | $i-C_4H_9$ |
| H | $s-C_4H_9$ |
| H | $t-C_4H_9$ |
| H | $CH_2CH=CH_2$ |
| H | $E-CH_2CH=CHCH_3$ |
| H | $CH_2C\equiv CH$ |
| H | $CH_2C\equiv CCH_3$ |
| H | Cyclopropyl |
| H | Cyclobutyl |
| H | Cyclopentyl |
| H | Cyclohexyl |
| $CH_3$ | $CH_3$ |
| $CH_3$ | $C_2H_5$ |
| $CH_3$ | $n-C_3H_7$ |
| $CH_3$ | $i-C_3H_7$ |
| $CH_3$ | $n-C_4H_9$ |
| $CH_3$ | $i-C_4H_9$ |
| $CH_3$ | $s-C_4H_9$ |
| $CH_3$ | $t-C_4H_9$ |
| $CH_3$ | $CH_2CH=CH_2$ |

| $R^1$ | $R^3$ |
|---|---|
| $CH_3$ | E-$CH_2CH=CHCH_3$ |
| $CH_3$ | $CH_2C\equiv CH$ |
| $CH_3$ | $CH_2C\equiv CCH_3$ |
| $CH_3$ | Cyclopropyl |
| $CH_3$ | Cyclobutyl |
| $CH_3$ | Cyclopentyl |
| $CH_3$ | Cyclohexyl |
| $C_2H_5$ | $CH_3$ |
| $C_2H_5$ | $C_2H_5$ |
| $C_2H_5$ | n-$C_3H_7$ |
| $C_2H_5$ | i-$C_3H_7$ |
| $C_2H_5$ | n-$C_4H_9$ |
| $C_2H_5$ | i-$C_4H_9$ |
| $C_2H_5$ | s-$C_4H_9$ |
| $C_2H_5$ | t-$C_4H_9$ |
| $C_2H_5$ | $CH_2CH=CH_2$ |
| $C_2H_5$ | E-$CH_2CH=CHCH_3$ |
| $C_2H_5$ | $CH_2C\equiv CH$ |
| $C_2H_5$ | $CH_2C\equiv CCH_3$ |
| $C_2H_5$ | Cyclopropyl |
| $C_2H_5$ | Cyclobutyl |
| $C_2H_5$ | Cyclopentyl |
| $C_2H_5$ | Cyclohexyl |
| n-$C_3H_7$ | $CH_3$ |
| i-$C_4H_9$ | $C_2H_5$ |
| n-$C_3H_7$ | n-$C_3H_7$ |
| i-$C_4H_9$ | i-$C_3H_7$ |
| n-$C_3H_7$ | n-$C_4H_9$ |
| s-$C_4H_9$ | i-$C_4H_9$ |
| i-$C_3H_7$ | s-$C_4H_9$ |
| t-$C_4H_9$ | t-$C_4H_9$ |
| i-$C_3H_7$ | $CH_2CH=CH_2$ |
| t-$C_4H_9$ | E-$CH_2CH=CHCH_3$ |
| i-$C_3H_7$ | $CH_2C\equiv CH$ |
| t-$C_4H_9$ | $CH_2C\equiv CCH_3$ |
| i-$C_3H_7$ | Cyclopropyl |
| t-$C_4H_9$ | Cyclobutyl |

| $R^1$ | $R^3$ |
|---|---|
| $i-C_3H_7$ | Cyclopentyl |
| $t-C_4H_9$ | Cyclohexyl |
| H | Benzyl |
| $CH_3$ | Benzyl |
| H | 4-Methylbenzyl |
| H | 4-Chlorbenzyl |
| H | 3-Methoxybenzyl |

Beispiel 1

Herstellung von 2-Amino-4-methylthio-6-trifluormethyl-1,3,5-triazin (V: $R^1$ = H, $R^3$ = $CH_3$)

Eine Suspension von 90,0 g (0,25 mol) N-Amidino-S-methylisothiuroniumiodid (Addukt an N-Methyl-2-pyrrolidon) in 128,0 g (1,0 mol) Trifluoressigsäuremethylester wurde bei 0 °C tropfenweise mit 90,0 g (0,5 mol) einer 30 gew.-%igen Lösung von Natriummethanolat in Methanol versetzt, worauf sich eine homogene, schwach gelbe Lösung bildete. Danach wurde das Reaktionsgemisch 5 Stunden bei 25 °C gerührt.

Die flüchtigen Anteile wurden bei 40 °C unter vermindertem Druck entfernt, der Rückstand kräftig mit 200 ml Wasser gerührt, der gebildete Feststoff abgesaugt und unter vermindertem Druck bei 50 °C getrocknet.

Man erhielt die Titelverbindung in einer Ausbeute von 94 % (Fp. 102 - 103 °C), als farblose Kristalle.

Beispiel 2

Herstellung von 2-Amino-4-methylthio-6-trifluormethyl-1,3,5-triazin (V: $R^1$ = H, $R^3$ = $CH_3$)

Eine Suspension von 90,0 g (0,29 mol) N-Amidino-S-methylisothiuroniumbromid (Addukt an N-Methyl-2-pyrrolidon) in 128,0 g (1,0 mol) Trifluoressigsäuremethylester wurde bei 0 °C tropfenweise mit 104,4 g (0,58 mol) einer 30 gew.-%igen Lösung von Natriummethanolat in Methanol versetzt, worauf sich eine homogene, schwach gelbe Lösung bildete. Danach wurde das Reaktionsgemisch 5 Stunden bei 25 °C gerührt. Die flüchtigen Anteile wurden bei 40 °C unter vermindertem Druck entfernt, der Rückstand kräftig mit 200 ml Wasser gerührt, der gebildete Feststoff abgesaugt und unter vermindertem Druck bei 50 °C getrocknet.

Man erhielt die Titelverbindung, die noch etwa 5 Gew.-% an N-Methyl-2-pyrrolidon enthielt, in einer Ausbeute von 90 %.

Beispiel 3

Herstellung von 2-Amino-4-methylthio-6-trifluormethyl-1,3,5-triazin (V: $R^1$ = H, $R^3$ = $CH_3$)

Eine Suspension von 20,0 g (55,6 mmol) N-Amidino-S-methylisothiuroniumiodid (Addukt an N-methyl-2-pyrrolidon) in 100 ml Essigsäureethylester und 6,2 g (61,4 mmol) Triethylamin wurde bei 5 - 15 °C tropfenweise mit 23,4 g (112,2 mmol) Trifluoracetanhydrid versetzt. Man rührte 15 h bei 25 °C, verdünnte den Ansatz mit 300 ml Methylenchlorid, wusch mit 2 x 100 ml Wasser, trennte die Phasen und trocknete die organische Phase über $Na_2SO_4$. Die flüchtigen Anteile wurden bei 40 °C unter vermindertem Druck entfernt und der ölige, blaßorange Rückstand kräftig mit 200 ml Wasser gerührt, worauf Kristallisation eintrat. Der gebildete Feststoff wurde abgesaugt und unter vermindertem Druck bei 50 °C getrocknet.

Man erhielt die Titelverbindung in einer Ausbeute von 80 %.

Beispiel 4

Herstellung von 2-Amino-4-methylthio-6-trifluormethyl-1,3,5-triazin (V: $R^1$ = H, $R^3$ = $CH_3$)

Eine Suspension von 54,0 g (0,25 mol) N-Amidinothioharnstoff (Addukt an N-Methyl-2-pyrrolidon) in 250 ml Methanol wurde bei 30 °C tropfenweise mit 31,5 g (0,25 mol) Dimethylsulfat versetzt. Die sich rasch bildende homogene Lösung wurde 3 Stunden bei 30 °C gerührt. Nach Zugabe von 32,0 g (0,25 mol) Trifluoressigsäuremethylester bei 25 °C wurden 54,0 g (0,30 mol) einer 30 gew.-%igen Lösung von Natriummethanolat in Methanol bei 0 °C zugetropft und 18 Stunden bei 20-25 °C gerührt. Man neutralisierte durch Zugabe von 4 N HCl, entfernte die flüchtigen Anteile unter reduziertem Druck bei 40 °C und rührte den Rückstand kräftig mit 200 ml Wasser. Das Produkt wurde abgesaugt, mit 200 ml Wasser gewaschen und unter reduziertem Druck bei 40 °C getrocknet.

Man erhielt die Titelverbindung in einer Ausbeute von 75 %, verunreinigt mit ca. 5 % des Folgeprodukts, 2-Amino-4-methoxy-6-trifluormethyl-1,3,5-triazin.

Beispiel 5

Herstellung von 2-Amino-4-methoxy-6-trifluormethyl-1,3,5-triazin (I: $R^1$ = H, $R^2$ = $CH_3$)

Eine Lösung aus (0,22 mol) 46,9 g 2-Amino-4-methylthio-6-trifluormethyl-1,3,5-triazin und 400 ml Methanol wurde bei 0 °C tropfenweise mit 40,2 g (0,22 mol) einer 30 gew.-%igen Lösung von Natriummethanolat in Methanol versetzt. Man rührte 2 h bei 0 °C und 65 Stunden bei 25 °C. Man neutralisierte durch Zugabe von 4N HCl, entfernte die flüchtigen Anteile unter reduziertem Druck bei 30 °C und rührt den Rückstand kräftig mit 100 ml Wasser. Das Produkt wurde abgesaugt und unter reduziertem Druck bei 40 °C getrocknet.

Man erhielt die Titelverbindung in einer Ausbeute von 85 % (Fp. 163 °C) als farblose Kristalle.

**Patentansprüche**

1. Verfahren zur Herstellung von Derivaten des 6-Trifluormethyl-1,3,5-triazins der allgemeinen Formel I

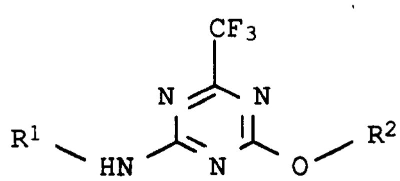

I

in der $R^1$ Wasserstoff oder einen Kohlenwasserstoffrest, der gegebenenfalls mit unter den Reaktionsbedingungen inerten Substituenten substituiert sein kann und $R^2$ einen Kohlenwasserstoffrest, der gegebenenfalls mit unter den Reaktionsbedingungen inerten Substituenten substituiert sein kann, bedeuten, dadurch gekennzeichnet, daß man ein Salz eines N-Amidino-S-alkylisothioharnstoffs der allgemeinen Formel II

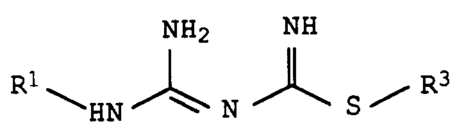

II

in der $R^3$ für einen Kohlenwasserstoffrest Rest mit 1 bis 10 C-Atomen, der gegebenenfalls mit unter den Reaktionsbedingungen inerten Substituenten substituiert sein kann, steht, mit einem Halogenid, Ester oder Anhydrid der Trifluoressigsäure (Verbindung III) sowie mit einer starken Base (IV) zu einem Derivat des 6-Trifluormethyl-1,3,5-triazins der allgemeinen Formel V

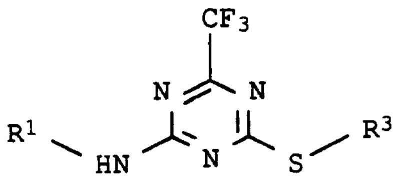

V

umsetzt und dieses in Gegenwart einer Base (VI) mit einem Alkohol der allgemeinen Formel VII

$R^2$-OH VII

in die Verbindung I überführt.

**2.** Verfahren zur Herstellung von Derivaten des 6-Trifluormethyl-1,3,5-triazins der allgemeinen Formel V

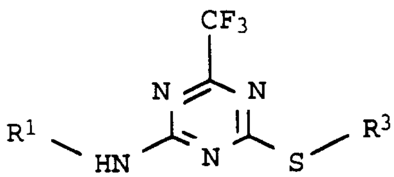

V

in der $R^1$ Wasserstoff oder einen Kohlenwasserstoffrest, der gegebenenfalls mit unter den Reaktionsbedingungen inerten Substituenten substituiert sein kann und $R^3$ einen Kohlenwasserstoffrest mit 1 bis 10 C-Atomen, der gegebenenfalls mit unter den Reaktionsbedingungen inerten Substituenten substituiert sein kann, bedeuten, dadurch gekennzeichnet, daß man ein Salz eines N-Amidino-S-alkylisothioharnstoffs der allgemeinen Formel II

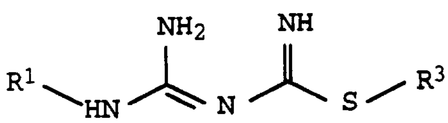

II

mit einem Halogenid, Ester oder Anhydrid der Trifluoressigsäure (Verbindung III) sowie mit einer starken Base (IV) umsetzt.

**3.** Verfahren zur Herstellung von Derivaten des 6-Trifluormethyl-1,3,5-triazins der allgemeinen Formel I

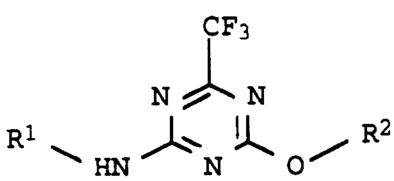

I

in der $R^1$ Wasserstoff oder einen Kohlenwasserstoffrest, der gegebenenfalls mit unter den Reaktionsbedingungen inerten Substituenten substituiert sein kann und $R^2$ einen Kohlenwasserstoffrest, der gegebenenfalls mit unter den Reaktionsbedingungen inerten Substituenten substituiert sein kann, bedeuten, dadurch gekennzeichnet, daß man eine Verbindung der allgemeinen Formel V

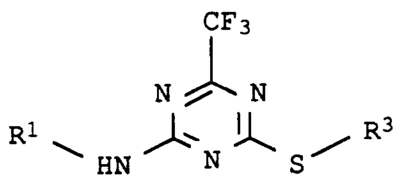

V

in der $R^3$ für einen Kohlenwasserstoffrest mit 1 bis 10 C-Atomen, der gegebenenfalls mit unter den Reaktionsbedingungen inerten Substituenten substituiert sein kann, steht, in Gegenwart einer Base (VI) mit einem Alkohol der allgemeinen Formel VII

$R^2$-OH VII

in die Verbindung I überführt.

**4.** Verfahren nach den Ansprüchen 1 bis 3, wobei die Substituenten $R^1$ bis $R^3$ die folgende Bedeutung haben:

$R^1$ Wasserstoff, eine $C_1$-$C_6$-Alkylgruppe, eine Cycloalkylgruppe mit bis zu 7 Kohlenstoffatomen oder Phenyl;

$R^2$ eine $C_1$-$C_4$-Alkylgruppe, eine $C_3$-$C_4$-Alkenylgruppe, eine $C_3$-$C_4$-Alkinylgruppe, eine $C_3$-$C_6$-Cycloalkylgruppe und

$R^3$ eine $C_1$-$C_4$-Alkylgruppe, eine $C_3$-$C_4$-Alkenylgruppe, eine $C_3$-$C_4$-Alkinylgruppe, eine $C_3$-$C_6$-Cycloalkylgruppe oder eine Benzylgruppe, die unsubstituiert oder mit Methyl, Chlor, Brom oder Methoxy kernsubstituiert ist.

## Claims

**1.** A process for the preparation of derivatives of 6-trifluoromethyl-1,3,5-triazine of the formula I

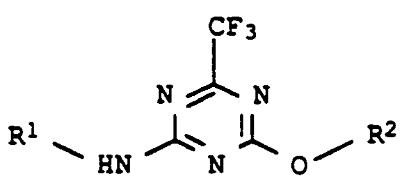

I

where $R^1$ is hydrogen or a hydrocarbon radical which may carry substituents which are inert under the reaction conditions and $R^2$ is a hydrocarbon radical which may carry substituents which are inert under the reaction conditions, wherein a salt of an N-amidino-S-alkylisothiourea of the formula II

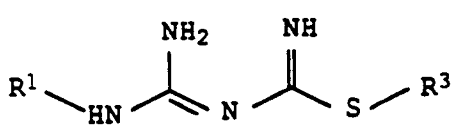

II

where $R^3$ is a hydrocarbon radical of 1 to 10 carbon atoms which may carry substituents which are inert under the reaction conditions, is reacted with a halide, ester or anhydride of trifluoroacetic acid (compound III) and with a strong base (IV) to give a derivative of 6-trifluoromethyl-1,3,5-triazine of the formula V

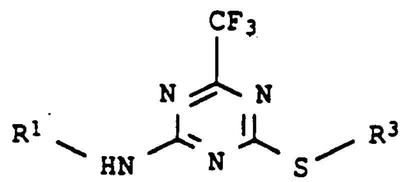

V

and the latter is converted in the presence of a base (VI) with an alcohol of the formula VII

$R^2$-OH VII

into the compound I.

2. A process for the preparation of derivatives of 6-trifluoromethyl-1,3,5-triazine of the formula V

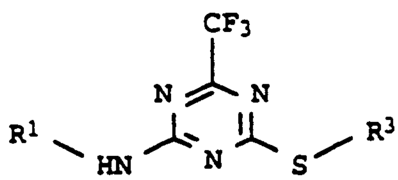

V

where $R^1$ is hydrogen or a hydrocabon radical which may carry substituents which are inert under the reaction conditions and $R^3$ is a hydrocarbon radical of 1 to 10 carbon atoms which may carry substituens which are inert under the raction conditions, wherein a salt of an N-amidino-S-alkylisothioureaof the formula II

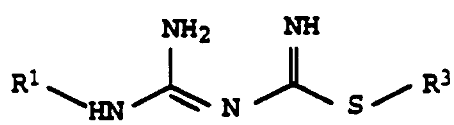

II

is reacted with a halide, ester or anhydride of trifluoroacetic acid (compound III) and with a strong base (IV).

3. A process for the preparation of derivatives of 6-trifluoromethyl-1,3,5-triazine of the formula I

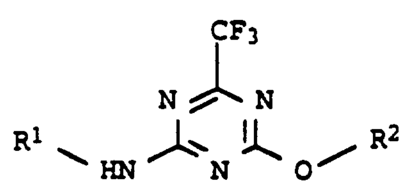

I

where $R^1$ is hydrogen or a hydrocarbon radical which may carry substituents which are inert under the reaction conditions and $R^2$ is a hydrocarbon radical which may carry substituents which are inert under the reaction conditions, wherein a compound of the formula V

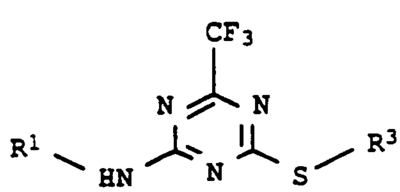

V

where $R^3$ is a hydrocarbon radical of 1 to 10 carbon atoms which may carry substituents which are inert under the reaction conditions, is converted in the presence of a base (VI) with an alcohol of the formula VII

$R^2$-OH VII

into the compound I.

4. A process as claimed in any of claims 1 to 3, wherein $R^1$ is hydrogen, $C_1$-$C_6$-alkyl, cycloalkyl of up to 7 carbon atoms or phenyl, $R^2$ is $C_1$-$C_4$-alkyl, $C_3$- or $C_4$-alkenyl, $C_3$- or $C_4$-alkynyl or $C_3$-$C_6$-cycloalkyl, and $R^3$ is $C_1$-$C_4$-alkyl, $C_3$- or $C_4$-alkenyl, $C_3$- or $C_4$-alkynyl, $C_3$-$C_6$-cycloalkyl or benzyl which is unsubstituted or substituted by methyl, chlorine, bromine or methoxy.

## Revendications

1. Procédé de préparation de dérivés de la 6-trifluorométhyl-1,3,5-triazine de formule générale I

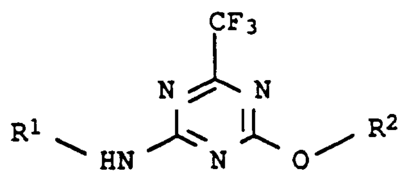

I

dans laquelle $R^1$ représente hydrogène ou un reste d'hydrocarbure éventuellement substitué par des substituants inertes dans les conditions de réaction, et $R^2$ représente un reste d'hydrocarbure éventuellement substitué par des substituants inertes dans les conditions de réaction, caractérisé par le fait que l'on fait réagir un sel d'une N-amidino-S-alkylisothiourée de formule générale II

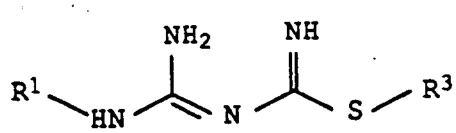

II

dans laquelle $R^3$ est mis pour un reste d'hydrocarbure de 1 à 10 atomes C, qui est éventuellement substitué par des substituants inertes dans les conditions de réaction, avec un halogénure, ester ou anhydride de l'acide trifluoracétique (composé III), ainsi qu'avec une base forte (IV), pour obtenir un dérivé de la 6-trifluorométhyl-1,3,5-triazine de formule générale V

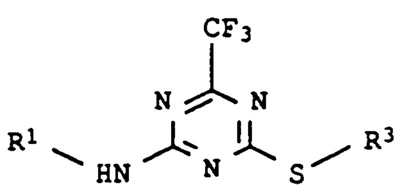

V

et on transforme celle-ci, en présence d'une base (VI), avec un alcool de formule générale VII

$R^2$-OH VII

en composé I.

2. Procédé de préparation de dérivés de la 6-trifluorométnyl-1,3,5-triazine de formule générale V

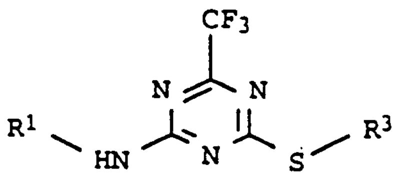

V

dans laquelle $R^1$ représente hydrogène ou un reste d'hydrocarbure éventuellement susbtitué par des substituants inertes dans les conditions de réaction, et $R^3$ représente un reste d'hydrocarbure de 1 à 10 atomes C, qui est éventuellement substitué par des substituants inertes dans les conditions de réaction, ce procédé étant caractérisé par le fait que l'on fait réagir un sel d'une N-amidino-S-alkylisothiourée de formule générale II

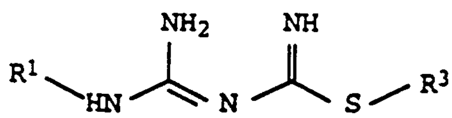

II

avec un halogénure, ester ou anhydride de l'acide trifluoracétique (composé III), ainsi qu'avec une base forte (IV).

3. Procédé de préparation de dérivés de la 6-trifluorométhyl-1,3,5-triazine de formule générale I

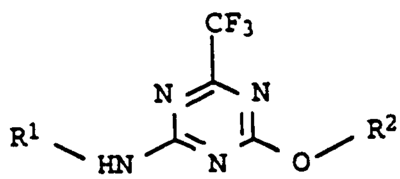

I

dans laquelle $R^1$ représente hydrogène ou un reste d'hydrocarbure éventuellement substitué par des substituants inertes dans les conditions de réaction, et $R^2$ représente un reste d'hydrocarbure éventuellement substitué par des substituants inertes dans les conditions de réaction, caractérisé par le fait que l'on transforme un composé de formule générale V

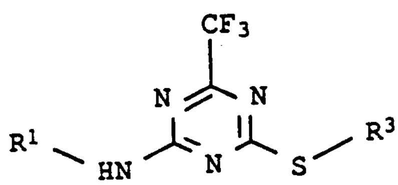

V

dans laquelle $R^3$ est mis pour reste d'hydrocarbure de 1 à 10 atomes C, qui est éventuellement substitué par des substituants inertes dans les conditions de réaction, en présence d'une base (VI), avec un alcool de formule générale VII

$R^2$-OH VII

en composé I.

**4.** Procédé selon les revendications 1 à 4 où les substituants $R^1$ à $R^3$ ont les significations suivantes :
$R^1$, hydrogène, un groupe alkyle en $C_1$-$C_6$, un groupe cycloalkyle ayant jusqu'à 7 atomes de carbone, ou phényle;
$R^2$, un groupe alkyle en $C_1$-$C_4$, un groupe alcényle en $C_3$-$C_4$, un groupe alcynyle en $C_3$-$C_4$, un groupe cycloalkyle en $C_3$-$C_6$ et
$R^3$, un groupe alkyle en $C_1$-$C_4$, un groupe alcényle en $C_3$-$C_4$, un groupe alcynyle en $C_3$-$C_4$, un cycloalkyle en $C_3$-$C_6$ ou un groupe benzyle non substitué ou substitué dans le noyau par méthyle, chlore, brome ou méthoxy.